# EUROPEAN PATENT SPECIFICATION

(11) **EP 0 970 953 B1**
(45) Date of publication and mention of the grant of the patent: **29.09.2004**
(21) Application number: 99112324.1
(22) Date of filing: 26.06.1999
(51) Int. Cl.: C07D 311/72

(54) **Manufacture of d,1-alpha-tocopherol**
Herstellung von alpha-Tocopherol
La préparation de l'alpha-tocophérol

(30) Priority: 10.07.1998 EP 98112842
(43) Date of publication of application: 12.01.2000
(73) Proprietor: DSM IP Assets B.V., 6411 TE Heerlen (NL)
(72) Inventor: Aquino, Fabrice, 68950 Reiningue (FR); Bonrath, Werner, 79115 Freiburg (DE)
(74) Representative: Schwander, Kuno Josef, Dr.

(56) References cited:
- EP-A- 0 694 541
- WO-A-97/28151
- I. V. KOZHEVINIKOV ET. AL.: "Syntheses of Vitamins E and K1 Catalysed by Heteropoly Acids" REACTION KINETICS AND CATALYSIS, vol. 47, no. 1, January 1992 (1992-01), pages 59-64, XP002113771 Budapest, HU

## Description

The present invention is concerned with a novel process for the manufacture of d,l-α-tocopherol by the acid-catalyzed condensation of trimethylhydroquinone (TMHQ) with isophytol (IP) or phytol (PH) in a solvent. As is known, d,l-α-tocopherol is a diastereoisomeric mixture of 2,5,7,8-tetramethyl-2-(4',8',12'-trimethyl-tridecyl)-6-chromanol (α-tocopherol), which is the most active and industrially most important member of the vitamin E group.

Many processes for the manufacture of d,l-α-tocopherol by the condensation of TMHQ with IP or PH in the presence of a catalyst or catalyst system and in a solvent or solvent system are described in the literature. These processes go back to the work of Karrer et al., Bergel et al. as well as Smith et al. [see Helv. Chim. Acta 21, 520 et seq. (1938), Nature 142, 36 et seq. (1938) and, respectively, Science 88, 37 et seq. (1938) and J. Am. Chem. Soc. 61, 2615 et seq. (1939)]. While Karrer et al. carried out the synthesis of d,l-α-tocopherol from TMHQ and phytyl bromide in the presence of anhydrous zinc chloride (ZnCl₂; a Lewis acid), not only Bergel et al. but also Smith et al. used TMHQ and PH as starting materials. In the following years mainly modifications, e.g. alternative solvents and Lewis acids, were developed. From the work of Karrer et al. there was developed in the year 1941 a technically interesting process for the manufacture of d,l-α-tocopherol which was based on the condensation of TMHQ with IP in the presence of the catalyst system ZnCl₂/hydrochloric acid (HCl) (US Patent 2 411 969). Later publications, e.g. Japanese Patent Publications (Kokai) 54380/1985, 64977/1985 and 226979/1987 [Chemical Abstracts (C.A.) 103, 123731s (1985), C.A. 103, 104799d (1985) and, respectively, C.A. 110, 39217r (1989)], describe this condensation in the presence of zinc and/or ZnCl₂ and a Bronsted (protonic) acid, such as a hydrohalic acid, e.g. HCl, trichloroacetic acid, acetic acid and the like, especially ZnCl₂/HCl, as the catalyst system. Disadvantages of these and further published processes featuring ZnCl₂ in combination with a Bronsted acid are the corrosive properties of the acids and the contamination of the waste water with zinc ions as a result of the large amount of ZnCl₂ required for the catalysis.

The manufacture of d,l-α-tocopherol by the reaction of TMHQ with phytyl chloride, PH or IP in the presence of boron trifluoride (BF₃) or its etherate (BF₃.Et₂O) is described in German Patents 960720 and 1015446 as well as in US Patent 3 444 213. However BF₃ too has corrosive properties.

Also, the condensation of TMHQ with IP or PH in the presence of a Lewis acid, e.g. ZnCl₂, BF₃ or aluminium trichloride (AlCl₃), a strong acid, e.g. HCl, and an amine salt as the catalyst system is described in European Patent Publication (EP) 100471. In an earlier patent publication, DOS 2606830, the IP or PH is pretreated with ammonia or an amine before the condensation with TMHQ in the presence of ZnCl₂ and an acid is effected. In both cases corrosion problems occur.

A further interesting method for the manufacture of d,l-α-tocopherol from TMHQ and IP comprises using an isolated TMHQ-BF₃ or -AlCl₃ complex and a solvent mixture featuring a nitro compound (DOS 1909164). This process avoids to a large extent the formation of undesired by-products because it involves mild reaction conditions. The yield of d,l-α-tocopherol, based on IP and the use of the solvent mixture methylene chloride/nitro-methane, is given as 77%. However, the use of such a solvent mixture is disadvantageous.

The manufacture of d,l-α-tocopherol by the condensation of TMHQ with IP using cation exchange resin complexes of metal ions (Zn²⁺, Sn²⁺ and Sn⁴⁺) is disclosed in Bull. Chem. Soc. Japan 50, 2477-2478 (1977); amongst other disadvantages it gives the product in unsatisfactory yields.

The use of macroreticular ion exchangers, e.g. Amberlyst® 15, as the catalyst for the condensation of TMHQ with IP is described in US Patent 3459773. However, the d,l-α-tocopherol could not be obtained in the requisite purity.

EP 603695 describes the manufacture of d,l-α-tocopherol in liquid or supercritical carbon dioxide by the condensation of TMHQ with IP or PH in the presence of acidic catalysts, such as ZnCl₂/HCl and ion exchangers. The reported yields are unsatisfactory.

The condensation in the presence of a catalyst system which consists of iron(II) chloride, metallic iron and HCl gas or aqueous solution is described in DOS 2160103 and US Patent 3789086. The formation of less by-products is advantageous compared with the aforementioned process using ZnCl₂/HCl. However, corrosion problems and chloride contamination are equally disadvantageous.

An interesting alternative for the condensation of TMHQ with IP to d,l-α-tocopherol comprises using trifluoroacetic acid or its anhydride as the catalyst (EP 12824). Although in this process the avoidance of HCl is achieved, the catalyst is relatively expensive.

The use of the heteropoly acid 12-tungstophosphoric or 12-tungstosilicic acid as the catalyst for the condensation of TMHQ with IP was described for the first time in React. Kinet. Catal. Lett. 47(1), 59-64 (1992). d,l-α-Tocopherol could be obtained, using various solvents, in about 90% yield.

A further process described in the literature [EP 658552; Bull. Chem. Soc. Japan 68, 3569-3571 (1995)] for the synthesis of d,l-α-tocopherol is based on the use of a scandium, yttrium or lanthanide fluorosulphonate, nitrate or sulphate, e.g. scandium trifluoromethanesulphonate. With up to about 10% excess of IP this process gives yields up to 98%.

The use of ion-exchanged bentonite, montmorillonite or saponite through treatment with e.g. scandium chloride and other metal salts (yttrium, lanthanum, etc.) as the catalyst for the condensation of TMHQ with IP or PH has as a disadvantage the need for a large amount of catalyst [EP 677520; Bull. Chem. Soc. Japan 69, 137-139 (1996)].

According to the Examples of EP 694 541 the condensation of TMHQ with IP to α-tocopherol can be achieved in high yields and with a high product purity when such solvents as carbonate esters, fatty acid esters and mixed solvent systems are employed, catalysis being effected by ZnCl₂/HCl. Disadvantages in this process are, in addition to the contamination of the waste water by zinc ions, the usual large "catalyst amount" of ZnCl₂ used.

According to WO 97/28151 the acid-catalysed condensation of TMHQ with IP can be performed in a cyclic carbonate or α-lactone as the solvent. The preferred catalyst is a mixture of ortho boric acid and oxalic, tartaric or citric acid, or boron trifluoride etherate.

From the forgoing explanations it is evident that most of the previously known processes have considerable disadvantages. Thus, corrosion problems occur in all processes in which such acid catalysts as boron trifluoride are used. Toxicity problems with the boron trifluoride adducts also occur, and when iron or zinc is used there is a contamination of the waste water with the metal ions which is today no longer acceptable. In some processes the formation of undesired by-products, e.g. phytyltoluene and chlorophytols, is an especially serious problem.

The object of the present invention is to provide a process for the manufacture of d,l-α-tocopherol by the condensation of trimethylhydroquinone with isophytol or phytol in the presence of a catalyst and in a solvent which does not have the disadvantages of previously known procedures. In this respect, it is necessary that the catalyst used has no, or at least a much reduced, corrosive action, is non-toxic, does not contaminate the environment and catalyzes the desired reaction as selectively as possible and in high yields. Furthermore, the catalyst should display its activity in small, really catalytic, amounts and should be readily separable and re-usable several times.

This object of the present invention is achieved by carrying out the condensation of trimethylhydroquinone with isophytol or phytol in the presence of at most 0.4 weight percent, based on the weight of isophytol or phytol, of 12-tungstophosphoric acid (H₃PW₁₂O₄₀), 12-molybdophosphoric acid (H₃PMo₁₂O₄₀) or 12-tungstosilicic acid (H₄SiW₁₂O₄₀) as the catalyst. Moreover, the condensation is effected in ethylene or propylene carbonate or a mixture of both carbonates, or in a mixture of one or both of the carbonates and a non-polar solvent, as the solvent or solvent system, as appropriate.

The condensation itself is represented in the following Reaction Scheme, showing the reaction with IP only.

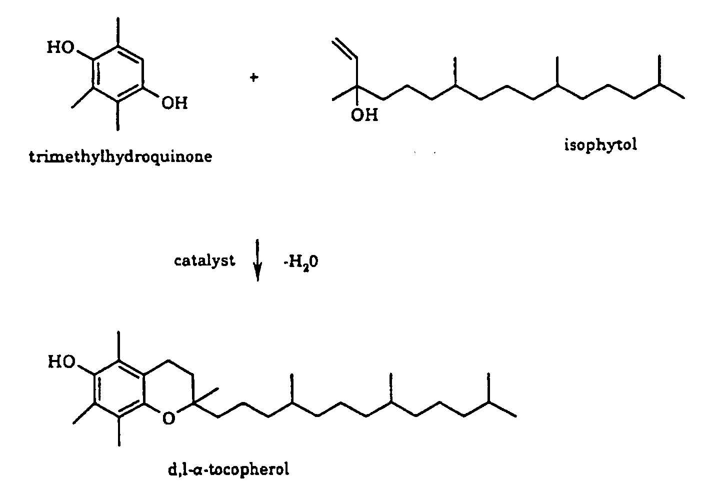

Accordingly, the process in accordance with the invention for the manufacture of d,l-α-tocopherol by the acid-catalyzed condensation of trimethylhydroquinone with isophytol or phytol in ethylene or propylene carbonate or a mixture of both carbonates, or in a mixture of one or both of the carbonates and a non-polar solvent, is characterized by carrying out the condensation in the presence of at most 0.4 weight percent, based on the weight of isophytol or phytol, of 12-tungstophosphoric acid, 12-molybdophosphoric acid or 12-tungstosilicic acid as the acid catalyst.

If in addition to ethylene or propylene carbonate or a mixture of both carbonates a non-polar solvent is employed, this is suitably hexane, heptane or octane, preferably heptane.

The condensation is conveniently effected at temperatures from 50°C to 150°C, preferably from 70°C to 130°C, especially at about 100°C.

Furthermore, trimethylhydroquinone is conveniently used in a molar excess of 30 to 120%, preferably 50 to 100%, over the amount of isophytol or phytol used. A particular range is 30 to 65% molar excess. The advantage of using a relatively large (up to 120%) molar excess of trimethylhydroquinone is that an efficient process operation involves a continuous recycling of the unreacted trimethylhydroquinone into the reaction medium.

The amount of acid catalyst is conveniently 0.1 to 0.4 weight percent, preferably about 0.35 weight percent, based on the weight of isophytol or phytol.

If the reaction is carried out in the presence of both a carbonate (ethylene or propylene carbonate, or both) and a non-polar solvent, then the volume ratio of the non-polar solvent to the carbonate used in the two-phase solvent system is conveniently in the range from 0.3:1 to 5:1, preferably from 1:1 to 3:2. The (total) amount of solvent, i.e. carbonate(s) and optionally also non-polar solvent, is such that conveniently 10 to 100 ml, preferably about 50 to 80 ml, for example about 30 to 60 ml, of carbonate(s) are used per 100 mmol of trimethylhydroquinone, and an additional 10 to 150 ml, preferably 25 to 100 ml, of non-polar solvent are used per 100 mmol of isophytol or phytol. In any event, only the one or the other carbonate is preferably used, either as the sole solvent or as the carbonate component of the solvent system with the non-polar solvent. As the latter, heptane is preferably employed, and the carbonate itself is preferably ethylene carbonate.

Moreover, the condensation is conveniently carried out under an inert gas atmosphere, preferably gaseous nitrogen or argon.

As the acid catalyst there is preferably used 12-tungstophosphoric acid or 12-tungstosilicic acid.

The process in accordance with the invention can be carried out operationally in a very simple manner by adding isophytol or phytol or a solution thereof in the optionally employed non-polar solvent dropwise to a solution or suspension of the trimethylhydroquinone and the acid catalyst in ethylene or propylene carbonate or a mixture of both carbonates. The rate at which the isophytol or phytol is added is not critical. Conveniently, however, it or the solution thereof is added dropwise over a period of 0.1 to 3, preferably 0.3 to 2.0, hours. After completion of the isophytol or phytol addition and an appropriate subsequent condensation, during which it is advantageous to remove the resulting water by azeotropic distillation or in the flow of inert gas used, isolation and purification of the obtained d,l-α-tocopherol can be effected by procedures conventionally used in organic chemistry, e.g. by distillation. The process can be carried out batchwise or continuously.

Particular advantages in the use of the acid catalyst in the process in accordance with the invention are, in addition to high yields of d,l-α-tocopherol, the avoidance of corrosion, the avoidance of waste water contamination with heavy metal ions, the high selectivity as well as the enabled ready isolation of the produced d,l-α-tocopherol from the mixture after reaction.

The process in accordance with the invention is illustrated by the following Examples:

### Examples 1-22

To a mixture of 23.3 g (150 mmol) of 2,3,5-trimethylhydroquinone (98% pure), 80 ml of ethylene carbonate or propylene carbonate (99% pure) and 150 mg of the heteropoly acid, 31.21 g (100 mmol) of isophytol (95% pure) either alone or in solution in up to 100 ml of heptane (or hexane or octane) were added dropwise under an argon atmosphere and with stirring at 70°C to 140°C, according to solvent, over a period of 20 to 120 minutes ("feed IP"). During the addition of isophytol an azeotropic mixture of water/heptane (or water/hexane or water/octane) was separated with the help of a water separator. After completion of the addition, the reaction mixture was heated under stirring at 140°C for another 30 minutes. The resulting two-phase system was cooled to 80°C and 100 ml of heptane (or hexane or octane) were added. The phases were separated and the carbonate layer was re-used. The heptane (or hexane or octane) layer was concentrated under reduced pressure to afford d,l-α-tocopherol as a brown oil. The results of the various trials are summarized in the following Tables 1 and 2.

**Table 1:**

| Condensation reaction between TMHQ and IP catalyzed by different heteropoly acids. | | | | | | |
|---|---|---|---|---|---|---|
| Ex. | solvent | catalyst (g/100 g product) | react temp. (°C) | feed IP (min.) | yield (%)^{a} | purity %)^{b} |
| 1 | EC | HPW (0.35) | 140 | 30 | 92.0 | 88.4 |
| 2 | EC | HPW (0.35) | 140 | 60 | 93.4 | 90.9 |
| 3 | EC | HPW (0.35) | 140 | 90 | 94.4 | 93.0 |
| 4 | EC | HPW (0.35) | 140 | 120 | 95.3 | 91.3 |
| 5 | PC | HPW (1.13) | 140 | 30 | 94.4 | 85.5 |
| 6 | EC+Hep | HPW (0.35) | 100 | 20 | 96.4 | 92.1 |
| 7 | EC+Hep | HPW (0.35) | 100 | 30 | 95.1 | 87.8 |
| 8 | EC+Hep | HPW (0.35) | 100 | 60 | 95.7 | 91.3 |
| 9 | EC+Hep | HPW (0.35) | 100 | 90 | 96.0 | 90.7 |
| 10 | EC+Hep | HPW (0.35) | 100 | 120 | 97.6 | 91.8 |
| 11 | EC+Hep | HSiW (0.35) | 100 | 20 | 96.6 | 91.8 |
| 12 | EC+Hep | HPMo (0.35) | 100 | 20 | 94.1 | 89.4 |

| | | | | | | |
|---|---|---|---|---|---|---|
| ^{a}isolated yield (not optimized, | | | | | | |
| ^{b}determined by gas-liquid chromatographical (GLC) analysis of the isolated products (int. standard), EC=ethylene carbonate, PC=propylene carbonate, Hep=heptane (in each case 100 ml used), HPW=H₃PW₁₂O₄₀, HSiW=H₄SiW₁₂O₄₀, HPMo=H₃PMo₁₂O₄₀. | | | | | | |

**Table 2:**

| Condensation reaction between TMHQ and IP catalyzed by HPW with different amounts of non-polar solvent (in each case feed IP = 30 min.) | | | | | |
|---|---|---|---|---|---|
| Ex. | non-polar solvent | ml^{*} | react. temp (°C) | yield (%)^{a} | purity (%)^{b} |
| 13 | - | 0 | 140 | 92.0 | 88.4 |
| 14 | hexane | 25 | 70 | 95.4 | 92.0 |
| 15 | hexane | 50 | 70 | 94.8 | 92.0 |
| 16 | hexane | 100 | 70 | 97.0 | 91.4 |
| 17 | heptane | 25 | 100 | 93.4 | 89.0 |
| 18 | heptane | 50 | 100 | 96.8 | 89.0 |
| 19 | heptane | 100 | 100 | 96.4 | 92.1 |
| 20 | octane | 25 | 127 | 93.0 | 85.7 |
| 21 | octane | 50 | 127 | 94.1 | 87.0 |
| 22 | octane | 100 | 127 | 93.2 | 83.9 |

| | | | | | |
|---|---|---|---|---|---|
| ^{*}based on 100 mmol TMHQ, | | | | | |
| ^{a}isolated yield (not optimized), | | | | | |
| ^{b}determined by GLC analysis of the isolated products (int. standard). | | | | | |

### Examples 23-28

To a mixture of 23.3 g (150 mmol) of 2,3,5-trimethylhydroquinone, 98% pure), 80 ml of ethylene carbonate (99% pure), 25 to 100 ml of hexane or heptane and 150 mg of 12-tungstophosphoric acid, 31.21 g (100 mmol) of isophytol (95%) were added dropwise under stirring at 70°C or 100°C over a period of 30 minutes. After completion of the addition, the reaction mixture was stirred at this temperature for another 30 minutes while removing the heptane (or hexane). The resulting two-phase-system was cooled to 80°C (or 60°C) and 100 ml of heptane (or hexane) were added. The phases were separated and the carbonate layer was re-used. The heptane (or hexane) layer was concentrated under reduced pressure to afford d,l-α-tocopherol as a brown oil. The results of the various trials are summarized in the following Table 3.

**Table 3**

| Condensation reaction between TMHQ and IP catalyzed by HPW whereby IP is added without solvent | | | | | |
|---|---|---|---|---|---|
| Ex. | non-polar solvent | ml* | react. temp. (°C) | yield (%)^{a} | purity (%)^{b} |
| 23 | hexane | 25 | 70 | 96.1 | 91.3 |
| 24 | hexane | 50 | 70 | 96.8 | 92.0 |
| 25 | hexane | 100 | 70 | 96.5 | 91.6 |
| 26 | heptane | 25 | 100 | 93.8 | 88.2 |
| 27 | heptane | 50 | 100 | 96.3 | 88.3 |
| 28 | heptane | 100 | 100 | 96.9 | 89.3 |

| | | | | | |
|---|---|---|---|---|---|
| * based on 100 mmol TMHQ, | | | | | |
| ^{a}isolated yield (not optimized), | | | | | |
| ^{b}determined by GLC analysis of the isolated products (int. standard). | | | | | |

## Claims

1. A process for the manufacture of d,l-α-tocopherol by the acid-catalyzed condensation of trimethylhydroquinone with isophytol or phytol in ethylene or propylene carbonate or a mixture of both carbonates or in a mixture of one or both of the carbonates and a non-polar solvent, which process is **characterized by** carrying out the condensation in the presence of at most 0.4 weight percent, based on the weight of isophytol or phytol, of 12-tungstophosphoric acid, 12-molybdophosphoric acid or 12-tungstosilicic acid as the acid catalyst.

2. A process according to claim 1, wherein the acid catalyst is 12-tungstophosphoric acid or 12-tungstosilicic acid.

3. A process according to claim 1 or 2, wherein the amount of acid catalyst is 0.1 to 0.4 weight percent, based on the weight of isophytol or phytol.

4. A process according to any one of claims 1 to 3, wherein the condensation is carried out in ethylene carbonate or a mixture thereof with a non-polar solvent.

5. A process according to any one of claims 1 to 4, wherein the non-polar solvent is hexane, heptane or octane, preferably heptane.

6. A process according to any one of claims 1 to 5, wherein the volume ratio of the non-polar solvent to carbonate in the two-phase solvent system is in the range from 0.3:1 to 5:1, preferably from 1:1 to 3:2..

7. A process according to any one of claims 1 to 6, wherein the condensation is effected at temperatures from 50°C to 150°C, preferably from 70°C to 130°C.

8. A process according to any one of claims 1 to 7, wherein trimethylhydroquinone is used in a molar excess over isophytol or phytol of about 30 to 120%, preferably 50 to 100%.

9. A process according to claim 8, wherein the molar excess is 30 to 65%.

10. A process according to any one of claims 1 to 9, wherein 10 to 100 ml, preferably 50 to 80 ml, of carbonate(s) are used per 100 mmol of trimethylhydroquinone and, if a non-polar solvent is also used, about 10 to 150 ml, preferably 25 to 100 ml, of the non-polar solvent are used per 100 mmol of isophytol or phytol.

11. A process according to claim 10, wherein 30 to 60 ml of carbonate(s) are used per 100 mmol of trimethylhydroquinone.

12. A process according to any one of claims 1 to 11, wherein isophytol or phytol or a solution thereof in the employed non-polar solvent is added dropwise to a solution or suspension of trimethylhydroquinone and the acid catalyst in ethylene or propylene carbonate or a mixture of both carbonates.

13. A process according to any one of claims 1 to 12, wherein the water resulting in the condensation is removed by azeotropic distillation or in the flow of inert gas used.

14. A process according to any one of claims 1 to 13, wherein the process is carried out batchwise or continuously.

## Patentansprüche

1. Verfahren zur Herstellung von d,1-α-Tocopherol durch die säurekatalysierte Kondensation von Trimethylhydrochinon mit Isophytol oder Phytol in Ethylen- oder Propylencarbonat oder einem Gemisch beider Carbonate oder in einem Gemisch von einem oder beiden der Carbonate und einem unpolaren Lösungsmittel, wobei das Verfahren durch Ausführen der Kondensation in Gegenwart von maximal 0,4 Gew.-%, bezogen auf das Gewicht von Isophytol oder Phytol, von 12-Wolframphosphorsäure, 12-Molybdänphosphorsäure oder 12-Wolframkieselsäure als Säurekatalysator, gekennzeichnet ist.

2. Verfahren nach Anspruch 1, wobei der Säurekatalysator 12-Wolframphosphorsäure oder 12-Wolframkieselsäure ist.

3. Verfahren nach Anspruch 1 oder 2, wobei die Menge an Säurekatalysator 0,1 bis 0,4, bezogen auf das Gewicht von Isophytol oder Phytol, ist.

4. Verfahren nach einem der Ansprüche 1 bis 3, wobei die Kondensation in Ethylencarbonat oder einem Gemisch davon mit einem unpolaren Lösungsmittel ausgeführt wird.

5. Verfahren nach einem der Ansprüche 1 bis 4, wobei das unpolare Lösungsmittel Hexan, Heptan oder Octan, vorzugsweise Heptan, ist.

6. Verfahren nach einem der Ansprüche 1 bis 5, wobei das Volumenverhältnis von dem unpolaren Lösungsmittel zu Carbonat in dem Zwei-Phasen-Lösungsmittelsystem im Bereich von 0,3:1 bis 5:1, vorzugsweise 1:1 bis 3:2, liegt.

7. Verfahren nach einem der Ansprüche 1 bis 6, wobei die Kondensation bei Temperaturen von 50°C bis 150°C, vorzugsweise 70°C bis 130°C, bewirkt wird.

8. Verfahren nach einem der Ansprüche 1 bis 7, wobei Trimethylhydrochinon in einem molaren Überschuss gegenüber Isophytol oder Phytol von 30 bis 120 %, vorzugsweise 50 bis 100 %, verwendet wird.

9. Verfahren nach Anspruch 8, wobei der molare Überschuss 30 bis 65 % ist.

10. Verfahren nach einem der Ansprüche 1 bis 9, wobei 10 bis 100 ml, vorzugsweise 50 bis 80 ml, Carbonat(e) pro 100 mMol Trimethylhydrochinon verwendet werden und, wenn ebenfalls ein unpolares Lösungsmittel verwendet wird, 10 bis 150 ml, vorzugsweise 25 bis 100 ml, des unpolaren Lösungsmittels pro 100 mMol Isophytol oder Phytol verwendet werden.

11. Verfahren nach Anspruch 10, wobei 30 bis 60 ml, Carbonat(e) pro 100 mMol Trimethylhydrochinon verwendet werden.

12. Verfahren nach einem der Ansprüche 1 bis 11, wobei Isophytol oder Phytol oder eine Lösung davon in dem angewendeten unpolaren Lösungsmittel tropfenweise zu einer Lösung oder Suspension von Trimethylhydrochinon und dem Säurekatalysator in Ethylen- oder Propylencarbonat oder einem Gemisch beider Carbonate zugegeben wird.

13. Verfahren nach einem der Ansprüche 1 bis 12, wobei das bei der Kondensation erhaltene Wasser durch azeotrope Destillation oder in dem verwendeten Inertgasstrom entfernt wird.

14. Verfahren nach einem der Ansprüche 1 bis 13, wobei das Verfahren chargenweise oder kontinuierlich ausgeführt wird.

## Revendications

1. Procédé de préparation du d,1-α-tocophérol par condensation catalysée à l'acide de triméthylhydroquinone avec de l'isophytol ou du phytol dans du carbonate d'éthylène ou de propylène ou un mélange des deux carbonates ou dans un mélange de l'un ou des deux carbonates avec un solvant non polaire, lequel procédé est **caractérisé par** la réalisation de la condensation en présence d'au plus 0,4 % en poids, sur la base du poids d'isophytol ou de phytol, d'acide 12-tungstophosphorique, d'acide 12-molybdophosphorique ou d'acide 12-tungstosilicique comme catalyseur acide.

2. Procédé selon la revendication 1, dans lequel le catalyseur acide est l'acide 12-tungstophosphorique ou l'acide 12-tungstosilicique.

3. Procédé selon la revendication 1 ou 2, dans lequel la quantité de catalyseur acide est de 0,1 à 0,4 % en poids sur la base du poids d'isophytol ou de phytol.

4. Procédé selon l'une quelconque des revendications 1 à 3, dans lequel la condensation est réalisée dans du carbonate d'éthylène ou un mélange de celui-ci avec un solvant non polaire.

5. Procédé selon l'une quelconque des revendications 1 à 4, dans lequel le solvant non polaire est l'hexane, l'heptane ou l'octane, de préférence l'heptane.

6. Procédé selon l'une quelconque des revendications 1 à 5, dans lequel le rapport volumique du solvant non polaire et du carbonate dans le système de solvant à deux phases est compris dans la plage allant de 0,3:1 à 5:1, de préférence de 1:1 à 3:2.

7. Procédé selon l'une quelconque des revendications 1 à 6, dans lequel la condensation s'effectue à des températures allant de 50°C et 150°C, de préférence de 70°C à 130°C.

8. Procédé selon l'une quelconque des revendications 1 à 7, dans lequel on utilise de la triméthylhydroquinone en excédent molaire de 30 à 120 %, de préférence de 50 à 100 %, par rapport à l'isophytol ou au phytol.

9. Procédé selon la revendication 8, dans lequel l'excédent molaire est de 30 à 65 %.

10. Procédé selon l'une quelconque des revendications 1 à 9, dans lequel on utilise de 10 à 100 ml, de préférence de 50 à 80 ml de carbonate(s) pour 100 mmol de triméthylhydroquinone, et, si un solvant non polaire est également utilisé, on utilise de 10 à 150 ml, de préférence de 25 à 100 ml, du solvant non polaire pour 100 mmol d'isophytol ou de phytol.

11. Procédé selon la revendication 10, dans lequel on utilise de 30 à 60 ml de carbonate(s) pour 100 mmol de triméthylhydroquinone.

12. Procédé selon l'une quelconque des revendications 1 à 11, dans lequel on ajoute au goutte-à-goutte de l'isophytol ou du phytol ou une solution de ceux-ci dans le solvant non polaire utilisé à une solution ou une suspension de triméthylhydroquinone et de catalyseur acide dans du carbonate d'éthylène ou de propylène ou un mélange des deux carbonates.

13. Procédé selon l'une quelconque des revendications 1 à 12, dans lequel l'eau provenant de la condensation est éliminée par distillation azéotropique ou dans le flux de gaz inerte utilisé.

14. Procédé selon l'une quelconque des revendications 1 à 13, dans lequel le procédé est réalisé de manière discontinue ou continue.
